# EUROPEAN PATENT APPLICATION

(11) **EP 3 650 032 A2**
(43) Date of publication of application: **13.05.2020**
(21) Application number: 18828213.1
(22) Date of filing: 04.07.2018
(51) Int. Cl.: A61K 35/42, A61K 31/192

(54) **COMPOSITION FOR RELIEVING AND TREATING BURNS AND BEDSORES**

(30) Priority: 07.07.2017 KR 20170086354
(71) Applicant: Lipobiolab Corporation, Chuncheon-Si, Gangwon-do 24232 (KR); Choi, Seong Hyun, Inje-gun, Gangwon-do 24610 (KR)
(72) Inventor: CHOI, Seong Hyun, Inje-Gun Gangwon-do 24610 (KR)
(74) Representative: Berggren Oy, Helsinki & Oulu
(86) International application number: PCT/KR2018/007599
(87) International publication number: WO 2019/009628

(57) **Abstract**

The present invention relates to a composition for relieving and treating burns and bedsores comprising the animal lung tissue extract as an active ingredient, wherein the composition of the present invention suggests that the protective effect of animal lung tissue extract and/or propionic acid on skin damage is related to not only inflammation inhibition but also regeneration and angiogenesis of fibrous tissues. It can be used as a therapeutic agent for these diseases by exhibiting industrially available effects related to the treatment of burns and bedsores.

## Description

### [Technical Field]

The present invention relates to a composition for alleviating and treating burns and bedsores.

### [Background Art]

Bed sores are conditions in which peripheral blood vessels of a tissue are blocked by pressure received from the contact surface of the body, causing necrosis of the tissue, and the healing is often required for a long time.

Therefore, long-term, long-term caregivers and long-term care patients tend to develop bedsores (so-called pressure ulcers), which is a major problem in nursing. In addition, bedsores are diseases that develop in animals other than humans, for example, saddle bedsores of horses are also one of the bedsores.

As a treatment for bedsores, ointments containing bromelain, bucladesine Na, but all the above-mentioned therapeutic agents are known to have side effects such as bleeding and pain.

In addition, the bed for beds, mattresses, etc. using a product to facilitate the ventilation of the bedsores is used, but this is only for the prevention and cannot be used as a therapeutic agent.

Therefore, most of the current bedsore treatment is treated depending on the natural healing power of the patient, a more safe method of bedsore treatment is required.

On the other hand, burns are mainly caused by an accident, and may be classified into burns by heat, burns by electric current, burns by chemical substances, burns by radiation, and the like, depending on the cause. The severity of the burn is divided into 1st, 2nd, 3rd and 4th degree burns depending on the width, depth of the burn, temperature and contact time of the object causing the burn, skin condition, etc. And from 2^{nd} degree burns require hospital treatment.

First-degree burns are accompanied by pain such as reddening of the skin and tingling. It causes damage to the epidermis, the outermost of the skin layers, and swells with pain and erythema. The symptoms disappear in a few days, but in some cases, desquamation and pigmentation remain. No scars remain after recovery. Sun burn is an example of the most common first-degree burns.

Second-degree burns affect both the epidermis and the dermis, causing erythema, pain, edema, and blistering within 24 hours of the accident. This burn also affects the gland and pores. Awakening is burning and painful. When the blisters burst, they show erosions and a large amount of secretion. Special care should be taken when the burned area reaches more than about 15% of the body surface area. Heals within a few weeks, but pigmentation and discoloration often remain in place. The secondary infection causes more local symptoms and lasts longer.

Third-degree burns affect the epidermis, dermis and hypodermis, making the skin black or translucent white and clotting blood vessels just below the surface of the skin. The burn may become numb, but the patient feels extreme pain, necrosis of the skin tissue and structure, which takes a lot of time to heal and scars. Two weeks after the accident, the scab peels off and the ulcer surface appears. There is a lot of secretion and easy to bleed, but gradually new tissue is formed, the epidermis regenerates, leaving scars and healing. In cases of deep skin necrosis or secondary infections, healing may be delayed and the surface of the scar may become irregular, resulting in keloids or deformations or movement disorders. Special care should be taken when the burned area covers more than 10% of the body surface area.

A fourth-degree burn is a case where the burned area tissue is carbonized and turned black, and a fat burn, ligament, fascia, muscle, and bone tissue located under the skin layer is burned. It occurs mainly in high-voltage electrical burns, and can sometimes occur when fungal infections occur in deep 2-3 degree burns. If the range of burn is more than 20%, a systemic physical reaction may occur, such as hypotension, shock, acute renal failure due to excessive fluid loss, and later wound infection, pneumonia, sepsis, multiple organ dysfunction syndrome, etc. may happen in some cases.

For the treatment of burns, it is important to heal the initial burn wounds or reduce the burn area as soon as possible. In early burn wound dressings, emphasis is placed on early treatments to control infection and inflammation, to maintain a moist environment, through the administration of growth factors or cytokines to help skin regeneration, and to use topical heparin to prevent growth into deep burns

Given the severity of such burn and/or pressure ulcer damage, development of therapeutic agents useful for the treatment or prevention of burn and/or pressure ulcer injury will greatly assist in the treatment, condition improvement and scar reduction of patients.

### [Prior Patent Document]

Korean Patent Publication No. 10-2008-0058126

### [Disclosure]

### [Technical Problem]

The present invention is derived to solve the above problems and to meet the above needs and an object of the present invention is to provide a novel bedsore treatment.
Another object of the present invention is to provide a novel burn treatment agent.

### [Technical Solution]

In order to achieve the above object, the present invention provides a composition for treating bedsores comprising an animal lung tissue extract as an active ingredient.

In one embodiment of the invention, the animal is preferably a pig, but is not limited thereto. In another embodiment of the present invention, the lung tissue extract is preferably a phospholipid fraction, but is not limited thereto.

In one embodiment of the present invention, the phospholipid fraction is preferable to be selected from the group consisting of phosphatidylinositol; 1-Palmitoyl-2-arachidonyl phosphatidylcholine; 1-palmitoyl-2-linoleoyl phosphatidylcholine; 1-palmitoyl-2-palmitoleonyl phosphatidylcholine; 1-palmitoyl-2-oleoyl phosphatidylcholine; dipalmitoyl phosphatidylcholine; phosphatidylethanolamine; and sphingomyelin or to include all of these phospholipids, but not always limited thereto.

In another embodiment of the present invention, the composition further preferably comprises an organic acid or a salt thereof, and more preferably, the organic acid is propionic acid, but is not limited thereto.

In another aspect, the present invention provides a composition for treating burns comprising an animal lung tissue extract as an active ingredient.

In one embodiment of the invention, the animal is preferably a pig, but is not limited thereto. In another embodiment of the present invention, the lung tissue extract is preferably a phospholipid fraction, but is not limited thereto.

In another embodiment of the present invention, the composition further preferably comprises an organic acid or a salt thereof, and more preferably, the organic acid is propionic acid, but is not limited thereto.

The concentration of the active ingredient in the bed sore treatment agent or prophylactic agent and burn treatment agent according to the present invention can be appropriately selected. Although treating or the prophylactic agent of bedsores or burn treatment according to the present invention may be administered as an oral agent, it is preferable to topically administer the wound, bedsore or burn to the affected area as an external preparation.

In addition, as a prophylactic agent for pressure sores, it is also possible to prevent bedsores from becoming difficult or less by topically administering them where there may be pressure sores.

The external preparation is not particularly limited as long as it is used as an external preparation, but for example, an ointment, a cream, a lotion, a liniment, a pap, a plaster, a patch, Warning agent, gel agent, liquid agent, tape agent, etc. are mentioned.

In the case where the formulation is an ointment or cream, a fat or oil base can be used as the base.

Examples of the oil-based base oil include hydrocarbons, higher alcohols, higher fatty acids, higher fatty acid esters, glycols, vegetable oils, and animal oils. The said oil-base can be used individually or in mixture of 2 or more types.

As said hydrocarbon, for example, C12-C32 hydrocarbon, fluid paraffin which is a mixture of various hydrocarbons, branched paraffin, solid paraffin, white petrolatum, yellow petrolatum, squalene, squalene, plasti-base, etc. can be mentioned. Among them, white petrolatum is particularly suitable. The said hydrocarbons can be used individually or in mixture of 2 or more types normally.

As said higher alcohol, a C12-30 aliphatic monohydric alcohol etc. are mentioned, for example. Specific examples of such aliphatic monohydric alcohols include lauryl alcohol, tridecyl alcohol, myristyl alcohol, pentadecyl alcohol, cetyl alcohol (cetanol), and hexadecyl alcohol, heptadecyl alcohol, stearyl alcohol, oleyl alcohol, oleyl alcohol, nonadecyl alcohol, eicosyl alcohol, seryl alcohol, melissyl alcohol, metostearyl alcohol, and cetostearyl alcohol. Among these, lauryl alcohol, cetyl alcohol and stearyl alcohol are preferable, and cetyl alcohol and stearyl alcohol are particularly preferable. The said higher alcohol can be used individually or in mixture of 2 or more types normally.

As said higher fatty acid, C6-C32 saturated or unsaturated fatty acid is mentioned, Specifically, for example, caproic acid (caproic acid), enanthic acid (enanthic acid), caprylic acid (caprylic acid), perargonic acid (pelargonic acid), capric acid (capric acid), undecyl acid, laurin Acid (lauric acid), tridecyl acid, myristylic acid, pentadecyl acid, palmitic acid, heptadecyl acid, stearic acid, oleic acid, nonadecanoic acid, arachnic acid, arachidonic acid, linoleic acid, linolenic acid, behenic aicd, lignoceric acid, cerotic acid, heptaconic acid, montanic acid, Melissic acid, laxelic acid, elaidic acid, brassidic acid, and the like are mentioned. Of these, palmitic acid and stearic acid are particularly preferable. The said higher fatty acid can be used individually or in mixture of 2 or more types normally.

Examples of the higher fatty acid esters include fatty acid esters such as myristyl palmitic acid, stearyl stearate, myristin myristyl, lignoserine seryl, serotonic acid laxeryl, laxelic acid laxeryl and the like; natural waxes from animals such as lanolin, beeswax, whale wax, shellac wax, etc., ester of fatty acids with 10 to 32 carbon atoms, and an aliphatic monohydric alcohol having 14 to 32 carbon atoms, natural waxes etc. from plants such as carnauba wax, candelila wax, etc.; ester, or their hydrogenated products of the saturated or unsaturated fatty acids in the carbon 10-22 and glycerol such as glyceryl monolaurylate, glyceryl monomyrilate, glyceryl monooleate, glyceryl monostearate, glyceryl dilaurylate, glyceryl dimyristyl Latex, glysyl distearate, glyceryl trilaurylate, glyceryl trimyrilate, glyceryl tristearate, etc. The said higher fatty acid ester can be used individually or in mixture of 2 or more types normally.

As said glycol, ethylene glycol, diethylene glycol, propylene glycol, 1, 3-butanediol, polyethylene glycol, etc. are mentioned, for example. These are used individually or in mixture of 2 or more types. Moreover, in this invention, it is preferable to mix and use the polyethyleneglycol of low polymerization degree and the polyethyleneglycol of high polymerization degree among these, for example. The said glycols can be used individually or in mixture of 2 or more types normally.

As said vegetable oil, for example, camellia oil, castor oil, olive oil, cacao oil, palm oil, palm oil, macadamia nut oil, soybean oil, tea seed oil, sesame oil, almond oil, safflower oil, cotton seed oil, terpene oil, and vegetable oils hydrogenated to these vegetable oils. The said vegetable oil can be used individually or in mixture of 2 or more types normally.

Examples of the animal oil include mink oil, yolk oil, squalane, squalene, lanolin, and derivatives of the animal oil. The said animal oil can be used individually or in mixture of 2 or more types normally.

Some of the higher alcohols may be used as absorption accelerators, and in the case of using such higher alcohols as a base, there is no need to specifically add an absorption accelerator. Examples of the oily base include oil in water (O/W) bases, water in oil (W/O) bases, suspension bases, and the like.

As the oil-in-water base, in the presence or absence of a surfactant, components such as lanolin, propylene glycol, stearyl alcohol, petrolatum, silicone oil, liquid paraffin, glyceryl monostearate, polyethylene glycol and the like is emulsified and dispersed base in an aqueous phase, and the like. The said base can be used suitably when preparing cream etc.

As said water-in-oil type base, the base etc. which emulsified and dispersed adding water in presence of a nonionic surfactant to components, such as petroleum jelly, a higher aliphatic alcohol, a liquid paraffin, etc. are mentioned.

The oil-in-water-based and water-in-oil-based bases are preferably used in water-containing formulations, such as water-containing solutions, lotions, pap agents, ointments, and the like.

Examples of the suspending base include an aqueous base obtained by adding a suspending agent such as starch, glycerin, high viscosity carboxymethyl cellulose, carboxy vinyl polymer, and the like to a gel phase.

The external preparation of the bedsore treatment agent or prophylactic agent and burn treatment agent which concerns on this invention can be manufactured by the preparation method of the external preparation generally employ adopted. For example, the ointment or cream agent may be prepared by mixing the base material by rolling mixing milling, emulsifying or suspending the base material according to each formulation, and then adding and mixing the active ingredient and various additives. In mixing, a generally used mixer such as a screw mixer, a homo mixer, a kneader, a roll mill, or the like can be used.

When the said formulation is a lotion, any type of suspension type, emulsion type, and solution type may be sufficient.

Examples of the suspension lotion include rubbers such as gum arabic and gum tragacanth, celluloses such as methyl cellulose, hydroxy ethyl cellulose and hydroxy ethyl starch, bentonite and non-gum. (veegum) a mixture of suspending agents of clays such as HV and the like, and the like. The base of the said suspension lotion can be used individually or in mixture of 2 or more types.

Examples of the base of the emulsion type lotion include bases emulsified with water and oil component such as fatty acids including stearic acid, behenic acid, oleic acid, and higher alcohols including stearyl alcohol, cetanol, behenyl alcohol, and the like. Can be. The base of the said emulsion type lotion can be used individually or in mixture of 2 or more types. Examples of the base of the solution type lotion include alcohols such as water, ethanol, glycerin, propylene glycol, and the like. The base of the said solution type lotion can be used individually or in mixture of 2 or more types normally.

The lotion may be prepared by, for example, adding various base ingredients to purified water, mixing and agitating the mixture, and then adding and mixing an active ingredient and an additive, and performing filtration as desired.

When the formulation is the liniment, as the base, for example, vegetable oils such as olive oil, sesame oil, almond oil, cotton seed oil, terepine oil, alcohols such as ethanol, propanol, isopropanol and a mixture of them and water, and the like. The base of the said liniment can be used individually or in mixture of 2 or more types normally.

The liniment may be prepared by dissolving an active ingredient in a base and then adding and mixing a desired component.

When the formulation is the pap, as the base, for example, water-soluble high molecular compounds such as polyvinyl alcohol, polyvinylpyrrolidone, polyacrylic acid and salts thereof, and the water-soluble high molecular compound is crosslinked with a polyvalent metal salt such as alum., the crosslinked body, such as which were crosslinked in the said water-soluble high molecular compound by the physical treatment like radiation irradiation, etc. can be mentioned. The base of the said pap can be used individually or in mixture of 2 or more types. The pap may be prepared by mixing an active ingredient, a base and a desired additive, and cooling after heating.

In the case of the plaster, patch and emplastrum agent, a support such as nonwoven fabric, elastomers such as dimethylpolysiloxane, styrene-isoprene-styrene rubber, isoprene rubber, natural rubber, styrene-butadiene rubber (SBR), butyl rubber, polyisobutylene, polyvinylalkyl ether, polyurethane, etc., fillers such as zincated, titanium oxide, silica, etc. adhesive agent such as terpene rosin, rosin resin or ester thereof, and phenol resin, exfoliation agents such as polyvinyl chloride, vinyl acetate, silicone resins, softening agents such as liquid paraffin, process oils, and anti-aging agents such as dibutylhydroxytoluene (BHT) can be included. These components can be used individually or in mixture of 2 or more types.

The plaster agent, patch agent, emplastrum agent, and the like can be produced by a common method such as solution method or hot pressure method.

Specifically, for example, in the case of a thermo-pressure type, the active ingredient and each component are uniformly roll mixed by a roll machine or the like, and applying on a release paper by using a calender with heat and pressure and coating so that it may become a uniform thickness and forming a drug containing layer, it can be manufactured by laminating , stacking and sticking on the support surface.

Also in the case of the said gel, liquid, tape, etc., the base should just be used for a normal external agent, and is not specifically limited.

Hereinafter, the present invention will be described in detail.

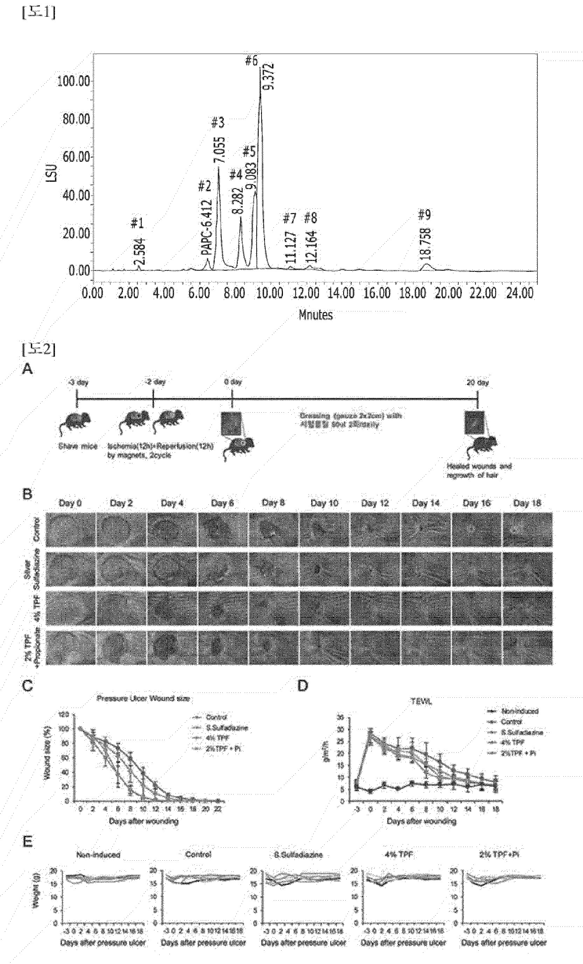

The composition for the treatment of bedsores and burns of the present invention is a total phospholipid fraction extracted by crushing the lung tissue of the animal and the DPPC content in the total phospholipid fraction is 25% or more (when the concentration is measured using HPLC-ELSD) and the main ingredient is shown in Figure 1, and it is characterized by selectively containing organic acid of microM concentration.

In the present invention, the total phospholipid fraction (TPF) is a phospholipid fraction extracted and purified from porcine lungs and a phospholipid surfactant present in the alveoli including dipalmitoyl phosphatidylcholine (DPPC) has strong polar head group and so can be easily mixed with water, and because it has two short saturated fatty acids in the molecule can act as a detergent (detergent).

In other words, by interfering with hydrophobic bonds and polar bonds that aggregate proteins at the same time to dissociate the blood serum proteins accumulated in the pressure sores to make them into smaller units, and facilitate vascular resorption, degradation by cells and external excretion of these proteins at the same time can be expected to play a role in reducing the water vapor by forming a polar fat film on the surface.

In addition, propionic acid (propionate, Pi), a short carbon chain organic acid with a carboxyl group, can interfere more effectively aggregation of proteins through bidirectional ionic bonds of various divalent cations such as calcium exuded in plasma.

In the experimental example of the present invention, as the bedsore treatment candidates, TPF and Pi is used, with the mouse animal model non-clinical evaluation to describe the bed sore treatment improvement and its mechanism.

In the present invention, it was shown that the treatment of TPF and TPF + Pi significantly inhibited the formation of ulcers after I/R. The recovery rate of wound area was increased by TPF dressing, skin moisture loss was improved by some TPF, and compared with silver sulfadiazine, which is widely used in the treatment of bedsores, effect of the composition of the present invention for inflammation inhibition, cell death inhibition, fiber tissue, blood vessel recovery and skin barrier improvement has been shown to be similar or more effective. In particular, low concentrations (2%) of TPF + Pi were similar to those of high concentrations of (4%) of TPF and showed better effects in reducing inflammation and cell death.

### [Advantageous Effects]

The composition of the present invention has the following effects on the treatment of burns and bedsores.

The present invention confirmed the TPF and Pi mediated protective mechanism of skin I/R damage. The results of the present invention indicate that treatment of TPF inhibits M1 macrophage invasion and inhibits dermatitis induced by proinflammatory mediators of M1 macrophages. In addition, it was confirmed that the treatment of low concentration TPF may have a similar effect to the high concentration TPF due to the addition of Pi.

The present invention showed that treatment of TPF and Pi reduced the cell number of I/R induced cell death. These results increase the phagocytosis and clearance of apoptotic cells after skin I/R and so prevents them from accumulating.

This shows suppression of apoptosis and necrosis and the protective effect of TPF and Pi from compression ulceration in I / R pressure beds.

In the present invention, it was confirmed that wound recovery of the bedsores was promoted and collagen content was increased by the treatment of high concentration TPF and low concentration TPF + Pi. In addition, confirming the rate of collagen type I / III production in the dermis, the treatment of TPF and Pi played an important role in the normal tissue recovery of skin bedsores after I/R. In addition, evaluating the vascular distribution of the I/R pressure sores, the number of perivascular cells in the I/R region was significantly higher than that of the control mice. The present invention suggests that the protective effect of TPF and Pi against skin I/R damage is related to the inhibition of inflammation as well as the regeneration of fibrous tissues and angiogenesis.

Based on the above results, the present invention suggests that TPF accelerates the pressure of decubitus healing and at the same time contributes to the tissue regeneration activity through anti-inflammatory, collagen production, angiogenesis, and skin barrier recovery, caused by skin I/R damage. It concludes that it suppresses the formation of pressure ulcers. In addition, it was proved that addition of Pi could show similar effects with high TPF even with low TPF. Ongoing treatment of TPF is shown to be effective and industrially useful as a material associated with skin wound healing, including decubitus ulcers and other ischemic ulcers.

### [Description of Drawings]

FIG. 1 is a diagram showing the phospholipid fraction extracted from pig lung tissue, the purified phospholipid fraction was analyzed using HPLC-ELSD system (Waters e2695 Waters 2424 (ELSD)). The column used water Xbridge column C18 5.0µm (4.6mm * 150mm). # 1, phosphatidylinositol; # 2, 1-Palmitoyl-2-arachidonyl phosphatidylcholine; # 3, 1-palmitoyl-2-linoleoyl phosphatidylcholine; # 4, 1-palmitoyl-2-palmitoleonyl phosphatidylcholine; # 5, 1-palmitoyl-2-oleoyl phosphatidylcholine; # 6, dipalmitoyl phosphatidylcholine; # 7 & # 8, phosphatidylethanolamine; # 9, sphingomyelin.
Fig. 2 is a diagram showing the effect of TPF and propionate on compression ulcer formation after skin I / R, (A) schematic of the wound healing model. After shaving the rat's dorsal skin and performing two I / R cycles. A control group (PBS) and test group (SS, 4% TPF, 2% TPF + Pi) were dressing twice a day and evaluated for bed sore for 20 days. (B) Pictures showing area of bedsores after I / R injury in C57BL / 6 mice. (C) Size change of bedsore area after I / R injury in C57BL / 6 mice. Mouse ulcer size after I/R induction was assigned to 100% values. (D) Wound area TEWL after I / R injury in C57BL / 6 mice. (E) Mice weight change after treatment with test group.

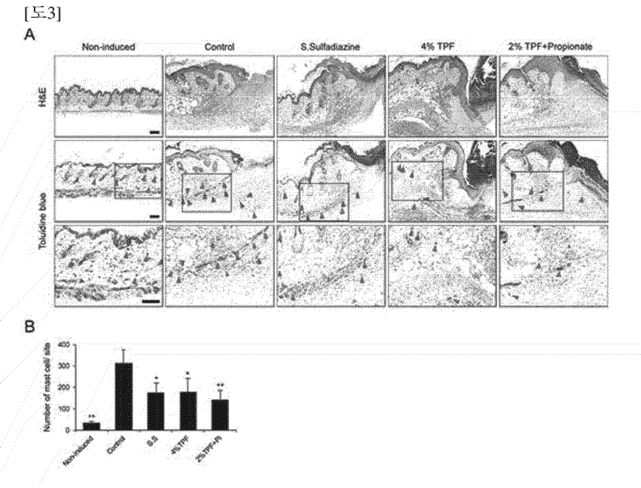

Fig. 3 shows the effect of TPF and propionate on the wound inflammatory response by skin I/R, (A and B) Tissue photograph of the wound 4 days after I / R injury in C57BL / 6 mice. (Red arrow: mast cell), Scale bar, 100µm.

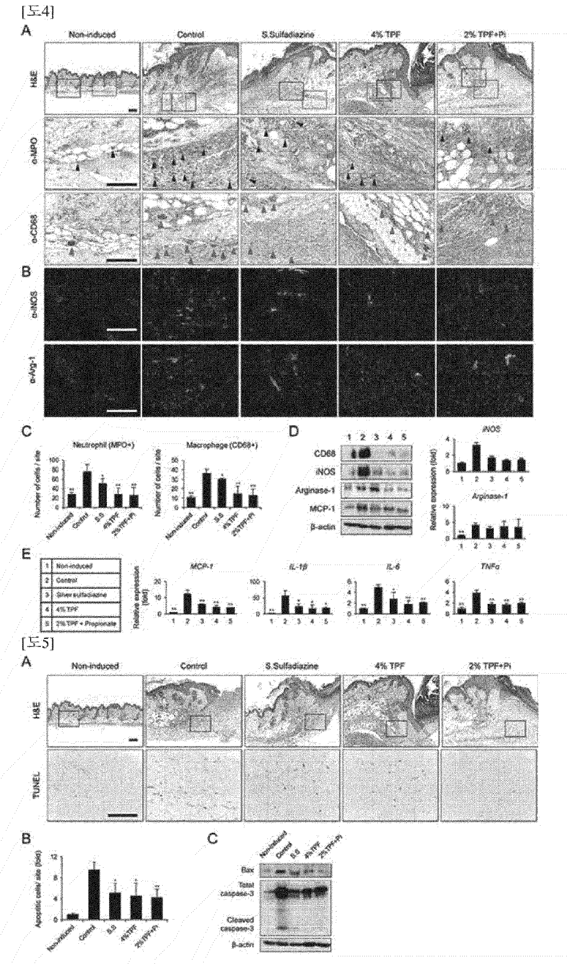

Fig. 4 shows the effect of TPF and propionate on the inhibition of macrophage infiltrating after I / R skin injury, (A) Tissue photographs of skin bedsores which dyed neutrophils and macrophages infiltrated with MPO and CD68 antibodies 4 days after I / R skin injury. (B) Tissue photographs of skin bedsores area stained with iNOS (M1 macrophages) and Arg-1 (M2 macrophage) antibodies. (C) Graph quantified by RT-PCR and immunoblot of expression levels of iNOS and arginase-1 4 days after I/R. (D) Picture quantifying mRNA expression levels of MCP-1, IL-1β, IL-6 and TNFα at 4 days after I/R. Scale bar, 100 µm. * p <0.05, ** p <0.01
Fig. 5 is a diagram showing the effect of TPF and propionate on the inhibition of apoptosis after I/R skin injury, (A) Histogram of skin bedsores using H & E and TUNEL staining 4 days after I / R injury in C57BL / 6 mice (B) Apoptotic cell counts in the skin sores 4 days after I / R injury. (C) Immunoblot quantifying expression levels of Bax and cleaved caspase-3 on 4 days after I/R. Scale bar, 100 µm. * p <0.05, ** p <0.01

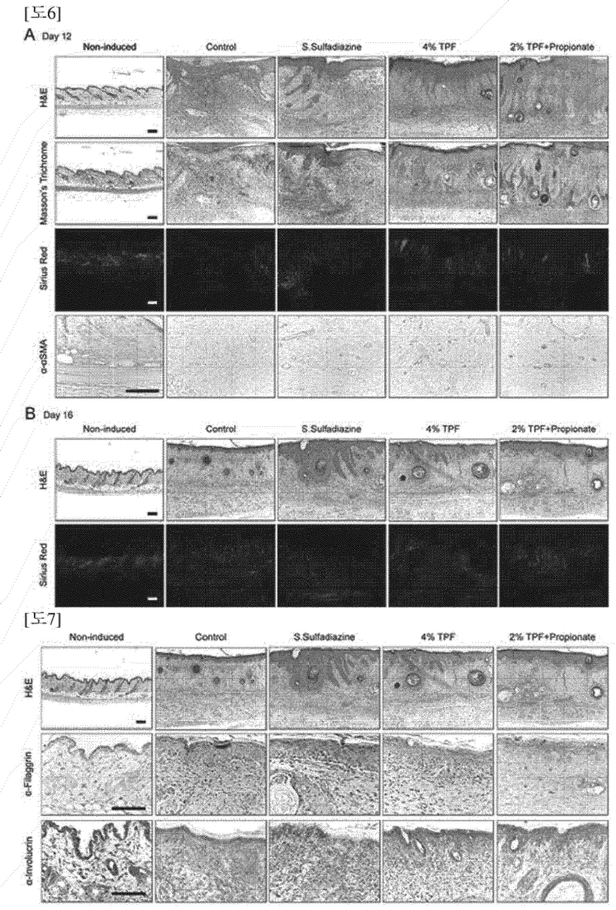

Fig. 6 shows the effects of TPF and propionate on the recovery of fibrous tissue after I/R skin injury. (A) Tissue photographs of skin bedsores area using H & E and masson's trichome, picro-sirius red, α SMA antibody staining 12 days after I / R injury in C57BL / 6 mice. (B) Tissue photographs of skin bedsores area using H & E and picro-sirius red staining 16 days after I / R injury in C57BL / 6 mice. Scale bar, 100 µm.
Fig. 7 shows the effect of TPF and propionate on skin barrier improvement after I/R skin injury. Histogram of skin bedsores using H & E, filaggrin, and involucrin antibody staining after 16 days of I / R injury in C57BL / 6 mice. Scale bar, 100 µm.

### [Mode for Invention]

Hereinafter, the present invention will be described in detail with reference to Examples. These examples are for illustrative purposes only and thus, are not interpreted to limit the scope of the present invention.

### Example 1. Formation of CCTF and MALDI analysis in dual target PCR

### Example 1: Purification of Phospholipid Fraction in Porcine Lung

Extraction method was used with method described in Moon et. al (Mol Biol Rep. 2012 39: 4237-4247).

Briefly, saline was added to fresh porcine lung tissue and crushed by a mixer, and CaCl2 was then added to a concentration of 5 mM to promote the precipitation of phospholipids in an aqueous crushed solution. The crushed sample was centrifuged to obtain a precipitate, and the precipitate was dissolved in an organic solvent of chloroform: methanol (1: 2) to obtain a total lipid fraction. The obtained total fat fraction was applied to an open silica column, eluted fatty acids and triglycerides were removed, and the total phospholipid fraction (TPF) was collected separately. The purified phospholipid fraction was concentrated using a vacuum rotary evaporator, and then freeze-dried at -70 degrees Celsius. Samples were stored at -20 ° C until use.

Since CaCl 2 solution was applied to easily precipitate the phospholipids in the extraction process we checked the presence or absence of calcium content in the final phospholipid fraction. Ca2 + detection was performed using Quantichrom ™ Calcium Assay Kit (DICA-500, BioAssy System, USA) and it was confirmed that all Ca2 + ions were removed during the organic solvent extraction process.

### Example 2: Preparation of Composition

The composition was prepared with adding the purified phospholipid fraction (TPF) extracted from porcine lung in 10 microM propionate / propionic acid (pH 5.8) solution. The final concentration of TPF was prepared to be 20 mg / ml.

### Experimental Example:

### 1) test material

Total phospholipid fraction (TPF), Propionate (propionic acid, Pi)

### 2) experimental method

### Test animals

Species and strain: C57BL / 6 (Female)
Source and Producer: Mice receive varieties produced through Central Experimental Animal (Seoul, Korea).
Acquisition and treatment of test substance: After 6 weeks of age, it is acclimated for 7 days to induce bedsores using magnets. The test substance (silver sulfadiazine, 4% TPF, 2% TPF + Pi) is then treated with dressing twice a day for 3 weeks.

### Grouping and test design

**[Table 1]**

| | Test group | Test substance | Treatment dosage | Treatment site | Treatment liquid volume |
|---|---|---|---|---|---|
| 1 | Normal(non-induction) | (-) | | dorsal | 50µl |
| 2 | Negative control | saline | | | |
| 3 | Positive control | Silver sulfadiazine | 10mg/ml | | |
| 4 | Test group 1 | 4% TPF | 40mg/ml | | |
| 5 | Test group 2 | 2% TPF+Pi | 20mg/ml | | |

Table 1 shows evaluation items, grouping and treatment dosage and volume.
Assessment of the recovery level of area inducing bedsores: visual evaluation, TEWL, weight change, wound size
Inflammatory response improvement assessment: mast cell, neutrophil, macrophage infiltration
Fibrous Recovery Assessment: Collagen Formation and I / III ratio
Assessment of Angiogenesis: Confirmation of aSMA expression
Evaluation of skin barrier improvement: Confirmation of filaggrin and involucrin expression evaluation of preventing cell death: Confirmation of apoptotic cell

### Induction of bedsores-like damage

Magnetic plate Ischemia / Reperfusion model (I/R). I/R periodic model has been previously reported. After anesthetizing the mouse, shave the back. Gently pull the skin and place it between the ceramic magnets with an average weight of 2.4g and 1,000G magnetic force (diameter, 12 mm; thickness, 5 mm). Transdermal and dermal tissue except muscle is located between the magnetic plates and ensure an average pressure of 2,000 mmHg. Repeat the ischemia and perfusion at 12 hour intervals and repeat twice. Evaluate according to each experiment schedule.

### Dressing

After the decubitus was induced, the decubitus was visually confirmed, and then the decubitus was first washed with physiological saline in both the experimental and control groups. The bedsores area was covered with a wet gauze dressing moistened with test material and then sutured using Tegaderm ™ (3M Health Care, St. Paul, MN, USA). Wrap once more with VetWrapTM (3M) for fixation. Dressing changes were performed twice a day (morning/evening) for three weeks.

### Visual observation evaluation of skin lesion

after evaluating the appearance of skin tissue damaged by ischemia, and then quantify it. The lesion site recovery was evaluated by the area during the observation period. Bedsores were measured by near visual photography (DSLR, Dermascope). Both experimental and control groups were visually confirmed on the second day of bed sores, and then photographed 10 times in three weeks. Eight data were used for analysis, except for some missing photographs and mice that were difficult to visually identify. The area of the bedsores photographed was calculated using imageJ software v1.44 (NIH, Bethesda, USA).

### Histopathological evaluation (H & E, Toluidine blue, Masson's trichrome, Sirius Red)

In order to observe the recovery of various skin tissues due to tissue damage, the skin tissues were separated from the mice on the 4th, 8th, 12th and 16th days after induction of bedsores and fixed with 10% paraformaldehyde (PFA) and then inserted into paraffin wax. Tissue sections were cut into 5 µm serial cross sections by microtome. Cut tissue is transferred to glass slides (Probe On PlusTM, Fisher Scientific, PA, USA), degreased, dehydrated. H & E (change of skin tissue), toluidine blue (mast cell infiltration), Masson's trichrome & Sirius Red (fibrous tissue recovery) staining was used for histological analysis. Each staining method was used to evaluate whether the test substance was involved in the reduction of inflammatory response, cell infiltration, and tissue recovery.

### Immunohistochemical staining

Inflammatory cells infiltrated into the skin tissue were separated, and specific antibodies for confirming skin barrier improvement were used to distinguish the nature of the infiltrated cells and confirmed changes in the skin. After Immune histochemical staining was performed using protein-specific markers involved in inducing ischemic damage, including inflammatory response improvement (MPO, CD68, iNOS, Arg-1), skin barrier improvement (Filaggrin, Involucrin), angiogenesis (αSMA), and cell death (TUNEL), the result of staining was observed and analyzed. Fluorescence images were obtained using Confocal microscopy (LSM 700, ZEISS, Jena, Germany).

### Immunoblot assay

The separated skin tissue was dissolved in PRO-PREP (iNtRON, Seongnam, Korea), and then centrifuged at 14,000 g for 20 minutes, and the supernatant was used for the experiment. Protein concentration was quantified using a BCA kit (Fisher Scientific, hampton, NH, USA). The separated supernatant (30 µg total protein) was transferred to PVDF membrane (Millipore, Danvers, MA, USA) after electrophoresis using 8-12% gel SDS-PAGE. After Blocking the transferred PVDF membrane in 5% skim milk powder for 1 hour, and then react the primary antibody (Bax, Cleaved caspase-3, GAPDH) with the membrane at 4 °C for 12 hours. After washing with TBST, the specific secondary antibody against the primary antibody was reacted at room temperature for 1 hour. After washing with Membrane, the result was evaluated using ChemiDoc ™ XRS + System (Bio-RAD, Hercules, CA, USA) after color development with ECL solution (Millipore).

### Observation of Inflammatory cytokine changes during healing

Expression changes of inflammatory cytokines gene involved in wound healing (MCP-1, IL-1β, IL-6, TNFα) were analyzed using real-time PCR. Total RNA was extracted from isolated skin tissue using TRIzol (Invitrogen, Carlsbad, CA, USA). Single stranded cDNA synthesis from the whole RNA template was performed with Prime ScriptTM RTmaster Mix (Takara, Tokyo, Japan). The resulting cDNA was subjected to real time PCR using CFX-96 (Bio-Rad, Hercules, CA, USA) using qPCR 2x PreMIX SYBR (Enzynomics, Seoul, Korea). PCR was used to amplify all genes, followed by 10 minutes of denaturation at 95 ° C, followed by 40 cycles (95 ° C 10 seconds, 60 ° C 15 seconds, 72 ° C 30 seconds). Expression data were calculated as cycle threshold (Ct) values using the ΔCt quantification method. Quantification was performed using GAPDH.

### Verification of statistical significance

The data of the present invention was expressed as "(mean ± standard deviation)", and the statistical analysis of each data was performed t-test according to the normality analysis. The significance level was determined as p <0.05 compared with the groups. (* p <0.05, ** p <0.01)

The results of the above experimental example are as follows.

### Evaluation of Protective Effects of TPF and Propionate on Bed Soreness after Cutaneous Ischemia / reperfusion-I / R in Mice

The effect of total phospholipid fraction (TPF) on bedsores and cutaneous pressure ulcer was examined. The wound area was compared after dressing with saline, silver sulfadiazine (SS), 4% TPF, and 2% TPF + Propionate (Pi) in the bedsores formed after I / R injury in C57BL / 6 mice. In the present invention, a simple, reproducible, non-invasive experimental mouse model was used to evaluate the pathogenesis of skin compression ulcers caused by I / R in vivo (Figure 2A). It was confirmed that the dressing treatment of test group added with 4% TPF significantly inhibited the formation of skin compression ulcer after I/R. In addition, the treatment of test group with Pi in the lower concentration of 2% TPF also suppressed the formation of skin compression ulcer at a level similar to that of 4% TPF. Comparing whether there is a difference in the initial bedside area of the bedside area caused by I / R, both the experimental group (SS, 4% TPF, 2% TPF + Pi) and the control group (Control) were formed to have similar areas, so the size of the bedsores before the experiment was confirmed to be the same (Fig. 2B). After 4 days of dressing treatment, the bedsore area of 4% TPF, 2% TPF + Pi treated mice showed a faster recovery to about 70-85% of the bedsore area of control mice (Figures 2B and C; SS). : 93%, 4% TPF: 75%, 2% TPF + Pi: 85%). However, the reduction of the initial bed sore area was somewhat lower in the 2% TPF + Pi-treated group, but after 6 days (25% and 26%, respectively), the reduction ratio tended to be similar. The size of the bedsore area of mice dressing with 4% TPF was significantly reduced after 4 days of dressing, and the size was significantly reduced after 6 days of mice dressing with 2% TPF + Pi, whereas wound closure time of control mice was longer than wound closure time after TPF treatment. Therefore, there was a significant difference in the rate of recovery of bedsores.

Next, the transepidermal water loss (TEWL) for the water vaporization of the wounded skin was measured (Fig. 2D). In the case of bedsores or burns, evaporation of water is increased when the function of the keratin is impaired or the keratin is completely absent. After measuring TEWL, the difference was not found at the beginning of dressing, but after 6 days when the wound size was recovered by more than 50%, skin moisture loss tended to decrease overall in the TPF dressing group compared with control group. In particular, no effect difference was observed between 4% TPF and 2% TPF + Pi. In addition, when the mouse was weighed to check the hazard of each test group, no abnormality was observed (Figure 2E). The results of this experiment show that TPF partially protects the formation of skin compression ulcers after I/R. Of these, high concentration (4%) TPF had a better effect on the initial recovery rate of bedsores, but afterwards, test group added with Pi to low concentration (2%) TPF showed similar recovery rate. The both test groups (4% TPF and 2%TPF+Pi) showed a better effect than SS group (positive group)

### Effects of TPF and Propionate on Inflammatory Responses of wound area after I/R Injury in Mice

Four days after I/R injury, histological changes were observed to observe infiltration of inflammatory cells. In conditions such as poorly cured compression ulcers, neutrophil infiltration, which contains ROS and various enzymes, leads to a chronic inflammatory state and The healing of these ulcers proceeds after the inflammation is controlled. Abnormal healing processes also lead to fibrosis, which often involves an increase in mast cells. In general, mast cells increase during wound healing and cause inflammation by releasing granules filled with enzymes, histamine and several active amines. Active amines released from mast cells cause peripheral blood vessels to leak, thus allowing monocytes to move quickly to the site of injury. In this experiment, toluidine blue staining was used to measure the degree of mast cell invasion of the bedsore-forming site and observed the inflammatory response (Figure 3).

In the control group, infiltration of mast cells was significantly increased by I/R skin injury. However, in the group treated with SS, 4% TPF and 2% TPF + Pi, the invasion of mast cells was significantly reduced (Figures 3A and B). and Statistically, the group treated with 2% TPF + Pi showed the best effect on the inhibition of invasion of mast cells. These results show that TPF reduces the inflammatory response by inducing a decrease in mast cell infiltration that mediates the inflammatory response and suggests that the effect is increased by the addition of Pi.

### Effect of TPF and Propionate on Macrophage Infiltration of wound site after I / R Injury in Mice

Neutrophils and macrophage are involved in I/R damage by regulating inflammation and angiogenesis. In this experiment, the effects of TPF and Pi on the infiltration and activity of neutrophils and macrophages after skin I/R injury were analyzed. Four days after dressing after I/R injury, edema of the dermis and inflammatory cell infiltration in the subcutaneous gland were observed histologically (Figure 4). In addition, the neutrophils and macrophages infiltrated after I/R using MPO and CD68 antibodies showed that the number of neutrophils and macrophages in the test group (SS, 4% TPF and 2% TPF + Pi) significantly reduced in comparison with the control group. This suggests that TPF may regulate the accumulation or function of neutrophils and macrophages, and that treatment of Pi with low concentrations of TPF (2%) is comparable to or higher than that of high concentrations (4%) of TPF (4%). Figures 4A and C). The local microenvironment in the skin promotes the development of classically activated macrophages (M1 macrophages) and alternatively activated macrophages (M2 macrophages). M1 macrophages are observed in early tissue damage responses, and inflammation is induced by the secretion of proinflammatory mediators including MCP-1, NO, IL-1, IL-6, IL-12 and TNFα. M2 macrophages, on the other hand, play an essential role in the early and intermediate stages, inducing inflammation reduction and promoting tissue repair. Therefore, this experiment examined the change of M1 and M2 macrophages at I/R site. We first identified the expression of iNOS (M1 marker) and Arg-1 (M2 marker) mRNA in each group (Figure 4C). Increased iNOS mRNA levels after I/R induction were significantly decreased in the groups treated with SS, 4% TPF and 2% TPF + Pi. However, Arg-1 mRNA levels tended to decrease slightly, but were not statistically significant. The iNOS and Arg-1 expression changes observed by immunoblot assay showed similar trends (Figure 4C). Then tissue infiltration was confirmed by tissue fluorescence staining. Early M1 and M2 macrophages are significantly increased during I/R injury. On the other hand, treatment with SS, 4% TPF and 2% TPF + Pi significantly decreased the total number of iNOS + M1 macrophages and slightly decreased Arg-1 + M2 macrophages (Figure 4B). However, when comparing the changes in iNOS and Arg-1 expression levels, the depletion of macrophages caused by TPF at early decubitus appears more concentrated in M1 macrophages. Next, The effect of TPF and Pi for mRNA levels of proinflammatory cytokines and chemokines MCP-1, IL-1β, IL-6, and TNFα, which can be secreted by M1 macrophages in I / R injuries was investigated using real-time PCR. SS, 4% TPF and 2% TPF + Pi dressings significantly inhibited the mRNA levels of proinflammatory cytokines and chemokines that increased with I / R damage (Figure 4D). There was no significant difference between 4% TPF and 2% TPF + Pi. This study suggests that 4% TPF and 2% TPF + Pi can reduce the tissue invasion of neutrophils and macrophages induced after I / R and alleviate skin inflammation. It is suggested that the addition of Pi at low concentration (2% TPF) can have a similar effect to the high concentration (4%) of TPF.

### Effect of TPF and Propionate on Inhibition of Apoptosis after I / R Injury in Mice

Reactive oxygen species (ROS) generated by I/R can induce apoptosis of skin cells and can cause an inflammatory response caused by secondary necrosis. TUNEL staining was performed on the skin tissue to confirm the effect of TPF and Pi on apoptosis of the skin damaged by I/R induction. The number of dead cells in the 4% TPF and 2%, TPF + Pi-dressed groups at the I / R induction site of day 4 skin tissues was reduced compared to the control mice (Figures 5A and B). In addition, changes in proteins involved in apoptosis were confirmed. Bax and cleaved caspase-3 are proteins that are activated when apoptosis occurs and are used as apoptosis markers. Treatment with 4% TPF and 2% TPF + Pi reduced the activity of Bax and cleaved caspase-3 induced upon I/R injury (Figure 5C). However, no significant difference was found between the two groups, but the addition of Pi at low concentration (2%) showed the effect of high concentration (4%) TPF. These results suggest that 4% TPF, 2% TPF + Pi can inhibit the formation and accumulation of apoptotic cells induced by cutaneous I/R damage.

### Effect of TPF and Propionate on fiber recovery and angiogenesis in wound site after I/R Injury in Mice

The effects of TPF and Pi on the recovery of fibrous tissues were examined 12 and 16 days after skin I/R injury. At day 12, epidermal regeneration was completed in all TPF-treated groups, but in part in the control and SS-treated groups, edema and bleeding were almost recovered (Figure 6A). Masson's trichrome and picro-sirius red were used to compare the collagen content in the tissue dermis and thereby the degree of recovery of the fibrous tissue was determined. Compared to the control group, the fibrilized tissue of the dermis was increased in all groups treated with SS, 4% TPF and 2% TPF + Pi. Normal dermis contains about 80% of type I collagen and 20% of type III collagen. In general, 24 hours after wounding, infiltrated fibroblasts begin to form new matrix by synthesizing and secreting type I and type III collagen. In granuloma tissue, type III collagen is much higher than normal tissue, but the percentage of type I collagen increases as the type I collagen is the main component and the closer to normal tissue is. We stained picro-sirius red on day 16 to see the rate of collagen production in the dermis after I/R induction (Figure 6B). In the control group treated with PBS only after I / R induction, the ratio of thin, green and yellowish type III collagen in the upper dermis was relatively high. However, in the test group dressing S.S., 4% TPF and 2% TPF + Pi, thicker, mature red and red-yellow type I collagen fibers with cross-linking, as seen in the core, were formed. This suggests that tissue remodeling is promoted later in the healing process of I/R-induced skin ulcers, leading to a rapid recovery to normal skin. However, no significant difference was observed between 4% TPF and 2% TPF + Pi, but the addition of Pi to low concentration (2%) of TPF suggests similar efficacy to treatment of high concentration (4%) of TPF.

In addition, the αSMA immunostaining was performed to observe the microvascular density during skin regeneration. The αSMA-positive microvascular density was significantly increased in the test group compared to the control group, and there was no significant difference between 4% TPF and 2% TPF + Pi (Figure 6A).

### Effect of TPF and Propionate on Skin Barrier Improvement after I/R Injury in Mice

Epidermal regeneration was not complete in all groups at 16 days after I/R injury, but almost all epithelial remodeling occurred. Immunohistochemical staining was performed to confirm the composition and condition of the recovered epithelium after I/R induction. As shown in Fig. 7, S.S., 4% TPF and 2% TPF + Pi increased the expression of filaggrin and involucrin, which are differentiation markers of epithelial cells, compared to the control group. Keratinocytes acquire epidermal protective functions by fundamentally changing their morphology and biochemical properties as the cells of the spinous layer finally differentiate from the granular layer to the stratum corneum. Insoluble keratinized keratin (K1, K10) is aggregated by the action of filaggrin to form a membrane-like structure on the layer called the keratin pattern to function to effectively maintain the water retention of the stratum corneum. In the cell membrane, a strong layered structure in which proteins effective for late keratinization processes such as involucrin and loricrin are bridged inside the cell membrane to form insoluble myocardium by forming various insoluble cell by the enzyme activity such as calcium-dependent transglutaminase. In order to complete the function as a protective film by reconstitution, the expression of them through differentiation after regeneration of the epidermal layer is important for the construction of normal epidermis. In the present invention, high (4%) TPF and low (2%) TPF + Pi dressings after I/R injury were effective in restoring normal skin by improving skin barrier by inducing keratinocyte differentiation as well as rapid epidermal regeneration.

## Claims

1. A composition for treating bedsores comprising an animal lung tissue extract as an active ingredient.

2. The composition for treating bedsores according to claim 1, wherein the animal is a pig.

3. The composition for treating bedsores according to claim 1, wherein the lung tissue extract is a phospholipid fraction.

4. The composition of claim 1, wherein the composition further comprises an organic acid or a salt thereof.

5. The composition for treating bedsores according to claim 4, wherein the organic acid is propionic acid.

6. A composition for treating burns comprising an animal lung tissue extract as an active ingredient.

7. The composition for treating burns according to claim 6, wherein the animal is a pig.

8. The composition for treating burns according to claim 6, wherein the lung tissue extract is a phospholipid fraction.

9. The composition of claim 6, wherein the composition further comprises an organic acid or a salt thereof.

10. The composition for treating burns according to claim 9, wherein the organic acid is propionic acid.
